(19) Europäisches Patentamt
European Patent Office
Office européen des brevets

(11) **EP 2 830 505 B1**

(12) **EUROPEAN PATENT SPECIFICATION**

(45) Date of publication and mention
of the grant of the patent:
**29.08.2018 Bulletin 2018/35**

(21) Application number: **13769560.7**

(22) Date of filing: **15.02.2013**

(51) Int Cl.:
**A61B 6/00** *(2006.01)*    *A61B 6/06 (2006.01)*

(86) International application number:
**PCT/US2013/026301**

(87) International publication number:
**WO 2013/148010 (03.10.2013 Gazette 2013/40)**

(54) **HYBRID PCI SYSTEM FOR MEDICAL RADIOGRAPHIC IMAGING**

HYBRIDES PCI-SYSTEM FÜR MEDIZINISCHE RÖNTGENBILDGEBUNG

SYSTÈME PCI HYBRIDE POUR IMAGERIE RADIOGRAPHIQUE À DES FINS MÉDICALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 US 201261617948 P**
**21.12.2012 US 201213724037**

(43) Date of publication of application:
**04.02.2015 Bulletin 2015/06**

(73) Proprietor: **Carestream Health, Inc.**
**Rochester, NY 14608 (US)**

(72) Inventors:
• **YIP, Kwok-leung**
**Rochester, NY 14608 (US)**
• **WOJCIK, Timothy, J.**
**Rochester, NY 14608 (US)**

• **SHAFER, Mark, E.**
**Rochester, NY 14608 (US)**
• **JADRICH, Bradley, S.**
**Rochester, NY 14608 (US)**
• **BATURIN, Pavlo**
**Rochester, NY 14608 (US)**

(74) Representative: **Wimmer, Hubert**
**WAGNER & GEYER**
**Gewürzmühlstrasse 5**
**80538 München (DE)**

(56) References cited:
**WO-A1-2011/122715    US-A1- 2007 183 560**
**US-A1- 2007 183 583    US-A1- 2009 092 227**
**US-A1- 2009 092 227    US-A1- 2010 246 764**
**US-A1- 2010 272 235    US-A1- 2011 085 639**

**Description**

**FIELD OF THE INVENTION**

The application generally relates to digital x-ray imaging methods/system, and more specifically, to methods and/or systems for acquiring multiple image information of an object (e.g., medical radiographic imaging) using a grating-based differential phase contrast imaging technique with a slot-scanning configuration.

**BACKGROUND OF THE INVENTION**

Conventional medical x-ray imaging devices are based on the attenuation through photoelectric absorption of the x-rays penetrating the object to be imaged. However, for soft tissues including vessels, cartilages, lungs, and breast tissues with little absorption, this provides poor contrast compared with bone images. This problem of low contrast in soft tissues can be addressed with phase contrast imaging (PCI) techniques.

The principle of PCI is based on the wave nature of x-rays, where refraction and diffraction properties need to be considered. As an electromagnetic wave, the x-ray is usually characterized by its frequency, amplitude, and phase. When an electromagnetic wave penetrates a medium, its amplitude is attenuated and its phase is shifted. In x-ray technology, the refractive index $n$ of a material can be expressed by a complex number

$$n = 1 - \delta + i\beta \qquad (1)$$

The imaginary part $\beta$ contributes to the attenuation of the amplitude and the real part $\delta$ is responsible for the phase shift. It has been shown that $\delta$ is about $10^3$ to $10^4$ times larger than $\beta$. But in conventional medical imaging, only the information of $\beta$ is recorded while the information of $\delta$ is completely lost. In recent years, several PCI techniques have been explored to make use of the phase shift to form the image, which is expected to provide more information about the object. These include (i) the interferometer technique, (ii) the diffraction-enhanced imaging (DEI) technique, and (iii) the free-space propagation technique.

However, there are various practical problems associated with all three techniques such as efficiency and limited field of view. In the case of perfect crystal interferometers and crystal diffractometers, high temporal coherence (i.e., a high degree of monochromaticity) is required; as a result, only a synchrotron or a well-defined wavelength of the whole spectrum from a radiation source is used. A synchrotron radiation source is costly and incompatible with a typical clinical environment. Both techniques are also limited by the accepted beam divergence of only a very small angle (a few mrad) due to the use of crystal optics. The free-space propagation technique is limited in efficiency since it requires high spatial coherence, which can only be obtained from an x-ray source with a very small focal spot. The three PCI techniques differ greatly in the way the image is recorded, the instrumental setup, and the requirements on the radiation source (especially its spatial and temporal coherence). Although some of the techniques yield excellent results for specific applications, none is very widely used and none has so far found application in medical diagnostics.

The grating-based PCI method with a standard x-ray tube is limited by the loss of visibility of the interference fringes at the detector due to the broad spectrum of the x-ray tube. A standard polychromatic x-ray tube generates soft x-rays (< 15 keV) that barely penetrate the skin at the low-energy portion of the spectrum, as well as hard x-rays (>50 keV) that penetrate through both bones and tissues at the high-energy portion of the spectrum. The use of an energy filter is thus preferred to obtain a narrow-bandwidth x-ray beam to reduce the radiation dose significantly by eliminating the unnecessary soft and hard x-rays and increase the clearness of the image.

For applications requiring a large FOV, a large-size phase grating G1 and analyzer grating G2 are needed. For example, a typical mammogram has a size of 24 cm x 30 cm. This means that a phase grating and an analyzer grating having the same size are required. Given the limitations of the current grating fabrication techniques (e.g., silicon wafer size, structure height, and grating uniformity), the manufacturing cost of such large gratings will be extremely high.

For a grating-based PCI system having a divergent cone beam (or fan beam) geometry and a large FOV, the phase contrast image quality is generally inferior in the edge regions of the detector. Toward the edges of plane gratings, the angle subtended by the grating bars with the incoming x-ray beam becomes larger. Since the bar height of the phase and analyzer gratings increase approximately linearly with the x-ray energy (E), the aspect ratio of bar height to gap width would be very large (> 10:1 for E > 20 keV). As a result, these gratings would cause a shadowing effect of the phase grating and the scan effect of the analyzer grating at larger angles, degrading the image quality.

Furthermore, US 2007 183 583 A1, relates to a focus-detector arrangement and an X-ray apparatus for generating projective or tomographic phase contrast recordings of a subject. The focus-detector arrangement includes a radiation source with a focus, arranged on a first side of the subject, for generating a fan-shaped or conical beam of rays. At least one X-ray optical grating is arranged in the beam path, with at least one phase grating arranged on the opposite second side of the subject in the beam path to generate an interference pattern of the X-radiation. An analysis-detector system detects at least the interference pattern generated by the phase grating in respect of its phase shift with position resolution. At least one X-ray optical grating includes bars which are free from overhangs that form shadows in the beam path of the fan-shaped or conical beam of rays.

US 2009 092 227 A1 discloses an interferometer for X-rays, for obtaining quantitative phase contrast images. The interferometer includes a standard polychromatic X-ray source, a diffractive optical beam splitter other than a Bragg crystal in transmission geometry, and a position-sensitive detector with spatially modulated detection sensitivity.

In all x-ray imaging systems, scattered radiation from the object has been shown to degrade the image quality in terms of subject contrast and contrast-to-noise ratio significantly. Currently, anti-scatter grid is the most widely used device for scatter rejection with most radiography and mammography systems. In mammography, with anti-scatter grid the amount of scattered radiation measured by the scatter-to-primary ratio can be reduced to between 0.1 and 0.3 from about 0.25 to 1.2. However, intrinsic to the anti-scatter grid method is the attenuation of a significant fraction of the primary x-rays.

## SUMMARY OF THE INVENTION

An aspect of this application is to advance the art of medical radiographic imaging.

Another aspect of this application to address in whole or in part, at least the foregoing and other deficiencies in the related art.

It is another aspect of this application to provide in whole or in part, at least the advantages described herein.

Another aspect of the application is to provide methods and/or apparatus embodiments for digital radiographic medical imaging. Another aspect of the application is to provide methods and/or apparatus embodiments for mammographic medical imaging. Another aspect of the application is to provide methods and/or apparatus embodiments for slot-scanning phase contrast imaging for large field of view (FOV) (e.g., greater than 100 mm square) radiographic medical imaging.

In accordance with the invention, a phase-contrast digital imaging system as set forth in claim 1 is provided. Further embodiments are inter alia disclosed in the dependent claims. The invention can inter alia provide a phase-contrast digital imaging system that can include a polychromatic x-ray source for radiographic imaging, a beam shaping assembly including a source grating G0 and a collimator configured to provide a long and narrow fan shaped x-ray beam having a beam length and a beam width. An x-ray grating interferometer comprises a phase grating G1, and an analyzer grating G2, wherein the phase grating G1 and the analyzer grating G2 are long and narrow and are covered by the beam length and the beam width. An area x-ray detector captures a radiographic image of an object placed between the source grating G0 and the phase grating G1. The x-ray detector has a long and narrow active area comprising an active length and an active width that are covered by the beam length and the beam width. The x-ray source, the source grating G0, the phase grating G1, the analyzer grating G2, and the detector are rigidly mounted to a swing arm. The swing arm is configured to pivot about an axis at the x-ray source to move the x-ray source, the source grating G0, the phase grating G1, the analyzer grating G2, and the detector about the axis to scan across the object while the object is in a stationary position. A pitch and a position of the analyzer grating G2 relative to a pitch of an interference pattern produced by the phase grating G1 produce at least one fringe pattern over a width of the analyzer grating G2.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

## BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.

FIG. 1 is a diagram that shows a side view of an exemplary embodiment of a scanning-slot phase contrast digital mammography imaging system according to the application.

FIG. 2 is a diagram that shows a functional block diagram of an embodiment of a slot-scanning grating-based phase contrast digital mammography imaging system as shown in FIG. 1.

FIG. 3 is a diagram that shows an exemplary embodiment of a slot-scanning grating-based phase contrast digital mammography imaging system according to the application.

FIG. 4 is a diagram that shows another exemplary embodiment of a slot-scanning grating-based phase contrast digital mammography imaging system according to the application.

FIG. 5 is a diagram that shows an embodiment of a long and narrow grating (e.g., formed by abutting two or more small gratings together) according to the application.

FIG. 6A is a diagram that shows a schematic of an exemplary three-grating phase contrast imaging system, and FIG. 6B is a diagram that shows a schematic of another exemplary three-grating phase contrast imaging system.

FIG. 7 is a diagram that shows intensity variation for one detector pixel (i, j) when one of the gratings (e.g., G2) is scanned along $x_g$ and the corresponding Fourier series coefficients.

FIG. 8 is a flow chart that shows a method embodiment for operating a slot-scanning grating-based phase contrast digital mammography imaging system according to the application.

FIG. 9 is a flow chart that shows another method embodiment for operating a slot-scanning grating-based phase contrast digital mammography imaging system according to the application.

FIG. 10 is a diagram that shows yet another exemplary embodiment of a slot-scanning grating-based phase contrast digital mammography imaging system according to the application.

FIG. 11 is a diagram that illustrates schematics for exemplary embodiments of tuned phase-contrast digital imaging systems and exemplary embodiments of detuned phase-contrast digital imaging systems.

FIG. 12 is a diagram that illustrates examples of the open field images measured in the detector plane for tuned and detuned configurations of phase contrast imaging system embodiments.

FIG. 13A is a diagram that shows several MTFs plotted for different alpha slopes, and FIG. 13B is a diagram that shows the percentage of the contrast drop as a function of MTF slope $\alpha$, spatial frequency f0 at 50% MTF drop, and the degree of the system detuning $\Delta f$.

FIG. 14 is a diagram that illustrates exemplary motion of interferometer with respect to objects or vise versa for a phase contrast imaging system embodiment.

FIG. 15 is a diagram that illustrates exemplary of object scan schematics that project individual slices of the object onto one-period modulated fringe pattern measured in the detector plane according to embodiments of the application.

FIG. 16 is a diagram that shows schematics of image formation mechanism that retrieves the intensity curves of individual slices of the scanned object, such as triangles, circles, and squares according to embodiments of the application.

## DESCRIPTION OF EXEMPLARY EMBODIMENTS

The following is a detailed description of exemplary embodiments according to the application, reference being made to the drawings in which the same reference numerals identify the same elements of structure in each of the several figures.

To be useful for clinical imaging, the phase contrast imaging systems must meet various requirements including: (i) use of a standard broadband x-ray source; (ii) a large field of view (FOV) of many centimeters (e.g., 24 cm x 30 cm for a typical mammography system); (iii) a reasonably compact design comparable to current radiographic imaging systems (e.g., the source-to-detector distance is about 65 cm for a typical mammography system); and/or (iv) a reasonable exposure time and dose (e.g., the mean exposure for a typical mammography system is about 5 mR).

1. System configuration

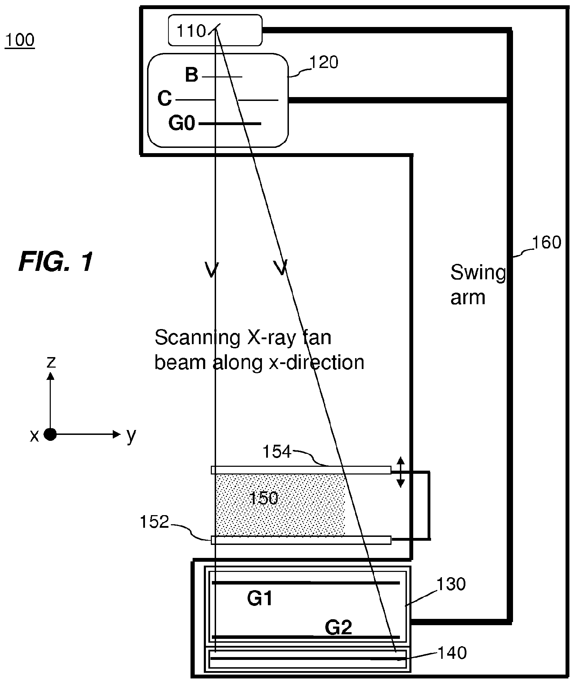

FIG. 1 is a diagram that shows an exemplary embodiment of a slot-scanning phase-contrast imaging system in accordance with the application. As shown in FIG. 1, a perspective view of a slot-scanning phase-contrast digital imaging system 100 can be used for mammography. The system 100 can include a conventional x-ray tube 110 for mammography imaging, a beam shaping assembly 120 comprising a filter or a tunable monochromator B, a collimator C, and a source grating G0, an x-ray grating interferometer 130 comprising a phase grating G1 and an analyzer grating G2, and an x-ray detector 140. The filter or a tunable monochromator B can be positioned after the collimator C. The three gratings (e.g., G0, G1, and G2) can be aligned in such a way that the plane and the grating bars of these gratings are parallel to each other. An object 150 (e.g., a breast) can be supported by a supporting plate 152 and is compressed by a compression paddle 154, which can be moved and adjusted (e.g., vertically).

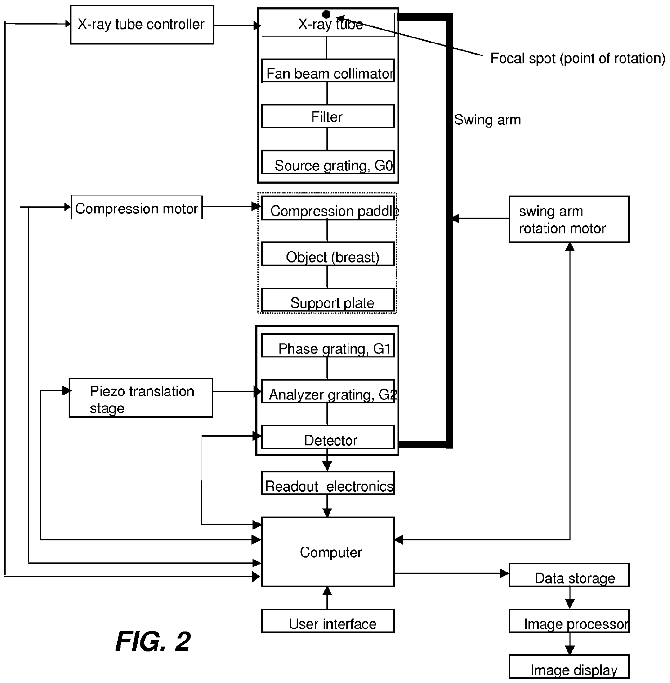

FIG. 2 is a functional block diagram that shows an exemplary embodiment of a slot-scanning phase-contrast imaging system. FIG. 2 shows a functional block diagram of the imaging system 100 used for mammography.

As shown in FIG. 1, the x-ray tube 110, the beam shaping assembly 120, the grating interferometer 130, and the detector 140 can move with a prescribed three-dimensional relationship to a radiation source. For example, the x-ray tube 110, the beam shaping assembly 120, the grating interferometer 130, and the detector 140 can be attached to a swing arm 160. The swing arm 160 can pivot around an axis co-axial with the focal spot of the x-ray tube 110. The x-ray tube 110 can be mounted at an angle with respect to the horizontal arm extension to illuminate an area of interest. The x-ray beam can be collimated to a narrow fan covering the interferometer 130 (e.g., gratings) and the active area of the detector 140 (e.g., about 24-cm long and 1-cm wide) by the collimator C. The entrance beam of the x-ray tube 110 can be slightly wider than the detector 140 and/interferometer 130 in order to reduce detector motion artifacts resulting from the edge of the detector 140 not being perfectly aligned with the collimator C at all times during the scan of an object.

2. System components

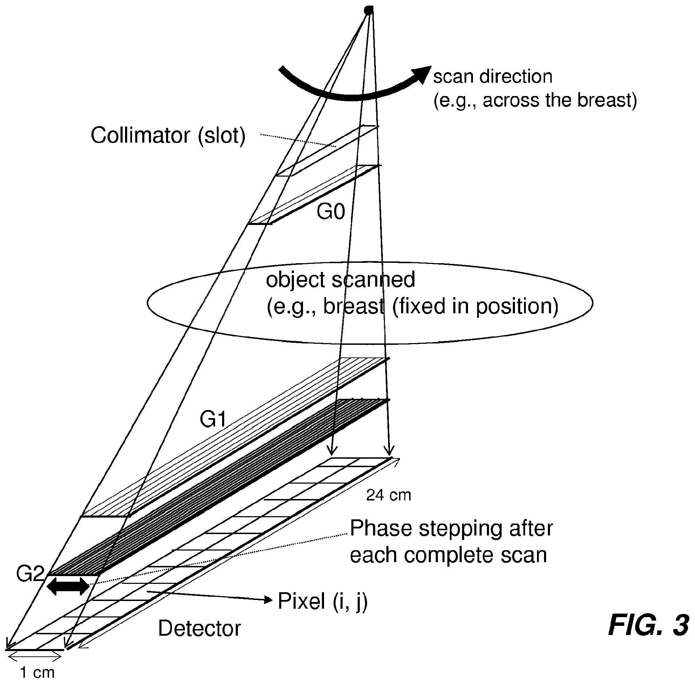

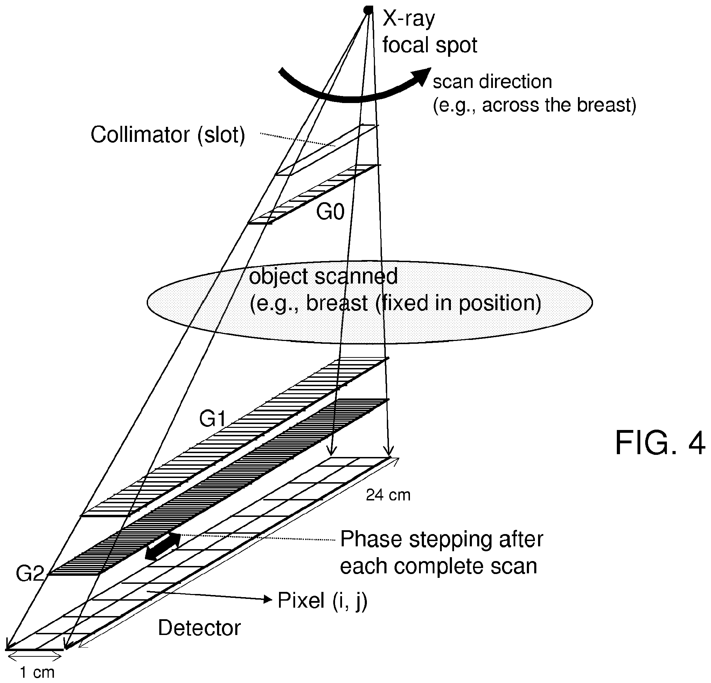

FIG. 3 is a diagram that shows a sectional illustration of an exemplary embodiment of components of a slot-scanning phase-contrast digital mammography imaging system in accordance with the application. FIG. 4 is a diagram that shows a sectional illustration of another exemplary embodiment of components of a slot-scanning phase-contrast digital mammography imaging system in accordance with the application. One difference between the imaging system of FIG. 3 and the imaging system shown in FIG. 4 is that the orientation of the grating bars of the gratings (e.g., the three gratings G0, G1, and G2) in FIG. 4 are parallel to the scan direction of the swing arm 160 (e.g., the x-ray fan beam), instead of being perpendicular to the scan direction of the swing arm 160 in FIG. 3.

(a) X-ray source

As shown in FIG. 1, the x-ray source 110 can be a conventional x-ray source. For example, the x-ray source 110 can be a polychromatic x-ray tube for mammography imaging. In this example, the x-ray source 110 can have a rotating anode made of tungsten (W), molybdenum (Mo), rhodium (Rh), or an alloy of heavy-element materials. The area of the focal spot can be between 0.01 mm$^2$ and 1.0 mm$^2$.

(b) Filter and monochromator

Beside inherent filtration associated with the x-ray tube 110, additional filtration (e.g., by the filter B) can be optionally used to spectrally shape the x-ray beam into a narrow-bandwidth beam to reduce or eliminate the unnecessary soft x-rays that are mostly absorbed by the patient and contribute to the radiation dose received during the examination, and/or the hard x-rays that can reduce the quality of the image. Exemplary typical filter materials are aluminum (Al), molybdenum (Mo), rhodium (Rh), silver (Ag), and other metals.

Alternatively, the filter B can be a tunable monochromatic x-ray filter that can be used with a divergent polychromatic x-ray source to produce monochromatic x-rays with a narrow spectrum centered at a selectable energy with a bandwidth of 1-3 keV.

(c) Gratings

As shown in FIG. 1, the imaging system 100 can include three gratings. In one embodiment, the source grating G0 can have absorbing gold bars, the phase grating G1 can be made of silicon, and the analyzer grating G2 can be made of absorbing gold bars. However, other materials can be used as know to one skilled in the art. The source grating G0 can be placed close to the x-ray source 110. The second grating G1 and the third grating G2 can have a fixed distance in between, for example, by being mechanically coupled together, electromechanically connected or rigidly coupled together. Similarly, the source grating G0 and the interferometer 130 can be coupled to have a variable, but known distance therebetween.

The source grating G0 can allow the use of a large incoherent x-ray source as the x-ray source 110 because the source grating G0 can create an array of individual line sources that each can provide sufficient spatial coherence for the interferometric contrast. The images created by the source grating G0 generated line sources can be superimposed congruently in the detector plane at the detector 140 leading to a gain in intensity (e.g., controllable interference).

The phase grating G1 can operate as a beam splitter and divide the incoming beam essentially into the $\pm$ 1 diffraction orders. These two $\pm$ 1 diffracted beams can interfere and form a periodic interference pattern in the plane of the second grating G2 through the Talbot self-imaging effect. When an object is inserted in the x-ray beam path, the position of the fringe pattern would change. As the change of the fringe position in the micron range is not determined with a common detector, an analyzer second grating G2 can be placed at a specific Talbot distance from the phase first gating G1 to enable the transform of fringe positions into intensity modulations on the detector 140 located directly behind the second grating G2 with the phase stepping technique.

As the source grating G0 is disposed close to the x-ray source 110 and the collimator C, the size the source grating G0 can be small (e.g., about 1 cm x 0.5 cm) because of the small angle subtended by the x-ray fan. For an exemplary (e.g., mammography) application, the FOV can be 24 cm x 30 cm. Since the object is located close to the interferometer formed by gratings G1 and G2, the size of these gratings should match the FOV. Given the state of art for standard photolithography techniques, repeatable fabrications of such large-area gratings G1 and G2 (e.g., 24 cm x 30 cm) with high or sufficient yield and an acceptable uniformity are not trivial. To address this fabrication problem, a standard 6 or 8 inch-silicon wafer can be used to fabricate multiple small gratings (e.g., each with an area of 8 cm x 1 cm) within a square of 8 cm x 8 cm. By abutting three pieces of small gratings together, a long and narrow grating (e.g., 24 cm x 1 cm) can be repeatedly obtained with acceptable uniformity.

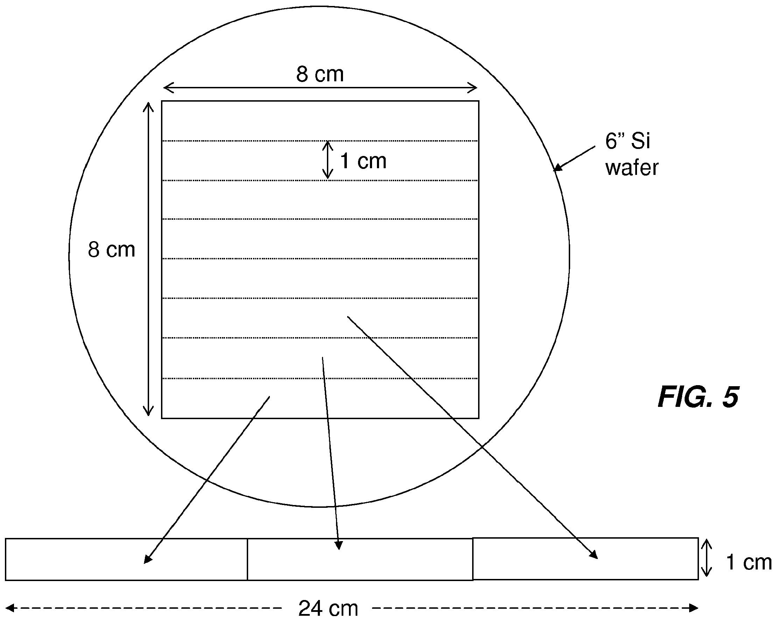

FIG. 5 is a diagram that shows an embodiment of a long and narrow grating (e.g., formed by abutting two or more small gratings together) according to the application. As shown in FIG. 5, one embodiment of the G1 grating or G2 grating can be formed using a standard silicon wafer. In one embodiment, a standard 8" wafer can be used to provide the long and narrow gratings G1 and G2.

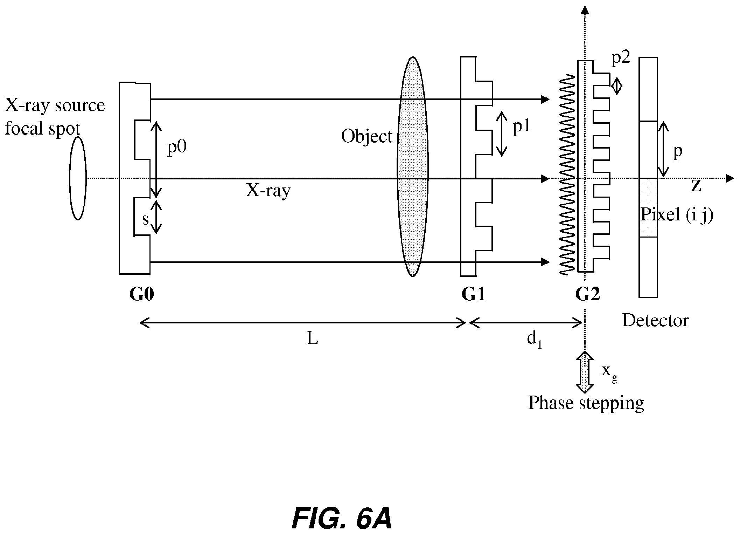

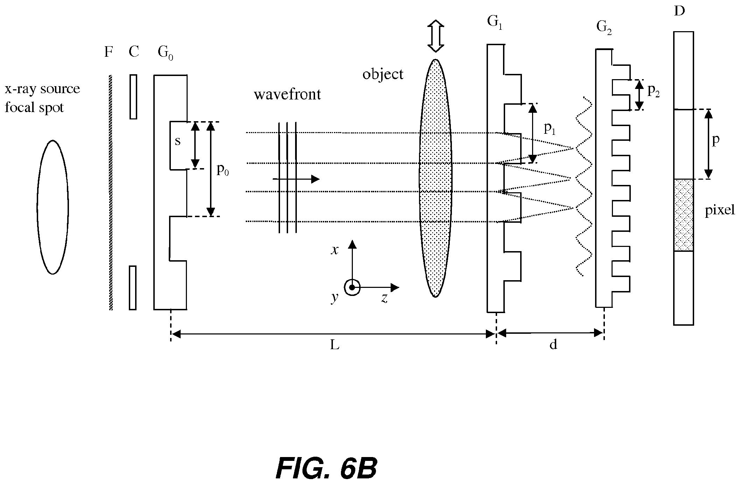

FIG. 6 is a diagram that shows a schematic of an exemplary three-grating phase contrast imaging system (e.g., interferometer). As shown in FIG. 6, three gratings, namely, the source grating G0 having absorbing gold bars, phase grating (or beam splitter) G1 made of silicon, and analyzer grating G2 having absorbing gold bars are used. The gratings are made from silicon wafers using standard photolithography techniques, and subsequently electroplating to fill the grooves with gold (G0 and G2). The interferometer is formed by G1 and G2. The plane and the grating bars of these three gratings are parallel to each other.

The source grating G0 allows the use of large incoherent x-ray sources since it creates an array of individual line sources each providing enough spatial coherence for the interferometric contrast. The images created by each line source are superimposed congruently in the detector plane leading to a gain in intensity. The phase grating G1 acts as a beam splitter and divides the incoming beam essentially into two first diffraction orders that interfere and form periodic fringe patterns in planes perpendicular to the optical axis (z). Based on the Talbot effect, the periodic fringe pattern, which is called the self image of the phase grating G1, will have its highest contrast at the first Talbot distance $d_1$ behind G1. Assuming that the phase shift undergone by x-rays passing through the grating bars of G1 is $\pi$, the first Talbot distance is given by

$$d_1 = \frac{p_1^2}{8\lambda} \qquad\qquad (2)$$

where $p_1$ is the period of G1 and $\lambda$ is the wavelength of x-ray for plane waves. The period of the fringe pattern ($p_2$) at the plane of the analyzer grating G2 placed at a distance of $d_1$ from G1 is approximately half the period of G1. The analyzer grating G2 has approximately the same period of the fringe pattern ($p_2$).

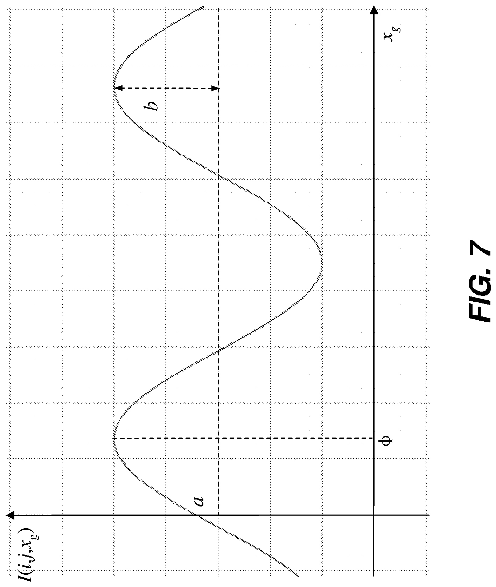

When an object is placed in the beam path, the incoming x-ray wavefront can be locally distorted by the object. Where the wavefront is distorted, the fringes formed by the phase grating G1 are displaced from their unperturbed positions. The fringe displacements are transformed into intensity variations by the analyzer grating G2 placed at a distance $d_1$ from the phase grating G1. This allows the use of an x-ray detector placed just behind the analyzer grating G2 with much larger pixels than the spacing of the fringes. Using the phase stepping technique, scanning the lateral position $x_g$ of one of the gratings over one period of the grating (here the analyzer grating G2) causes the recorded signal in each pixel to oscillate as a function of $x_g$ as shown in FIG. 7. FIG. 7 is a diagram that shows intensity variation for one detector pixel (i, j) when one of the gratings (e.g., G2) is scanned along $x_g$ and the corresponding Fourier series coefficients a, b, and $\phi$. The phase $\phi$ of the oscillation in each pixel is a measure of the wavefront phase gradient, while the average detector signal $a$ in each pixel over the grating scan is equivalent to the conventional absorption image. The total phase shift of the object can thus be retrieved by a single one-dimensional integration along the direction x.

FIG. 6B is a diagram that shows a schematic of another exemplary three-grating phase contrast imaging system. As shown in FIG. 6B, a three-grating PCI system can include stationary G0, G1, and G2 gratings and an object to be imaged can be moved (e.g., across) relative to the stationary G0, G1, and G2 gratings. In FIG. 6B, F is optional additional filtration and C is an optional collimator or beam shaping apparatus.

(d) Detector

For the detector 140, either an indirect or a direct flat-panel x-ray detector can be used. An indirect flat panel detector can include a layer of scintillator made of CsI, $Gd_2O_2S$, or other scintillating phosphors coupled with an array of photodiodes (e.g., a-Si photodiodes) and switches (e.g., thin-film transistor (TFT) switches). The thickness of the scintillator layer can be between 80 um and 600 um. The pixel pitch of the detector is ranged from 20 to 200 um. On the other hand, a direct detector can include a photoconductor such as amorphous selenium (a-Se) or $PbI_2$ to produce electrical charges on the detection of an x-ray. The electromagnetic radiation detection process is considered direct because the image information is transferred from x-rays directly to electrical charges with no intermediate stage.

As an alternative to the flat-panel detectors, a charge-coupled device (CCD) based x-ray detector can be used as the detector 140. For example, the CCD based x-ray detector can include a scintillating screen.

For a slot-scanning system, a tiled CCD detector array operated in time delay integration (TDI) mode is preferred to enable continuous scanning motion and x-ray illumination during each scan. The detector array can be formed by tiling two or more CCD devices together and can be coupled to a scintillator layer and a fiber optic plate (FOP). The FOP is used to protect the CCD array from radiation damage.

A slot-scanning system with a beam width comparable to the pixel width would require an extremely high tube output. The TDI operating mode of the CCD can allow a significantly wider beam to be used. The detected x-rays are first transformed into light photons via the scintillator layer. The light photons are then transmitted to the CCD through the FOP producing electrons in the CCD in response to the light emission from the scintillator upon x-ray absorption. By moving the electronic charges from pixel-to-pixel across the CCD width (e.g., columns), in synchrony with (e.g., at the same velocity) but in the opposite direction of the scanning motion, the TDI mode can enable x-ray integration across the CCD width while maintaining the pixel resolution. When the charges reach the last row of the CCD, the accumulated charge is read out and digitized. For example, the detector array can include four CCDs, each having a size of 6 cm x 1 cm, abutted along their narrow dimension to form a long and narrow detector (e.g., 24 cm x 1 cm). Again, the typical pixel size is between 20 um and 200 um.

As another alternative to the flat-panel detectors, a linear photon counting gaseous detector using avalanche amplification process can be also used as the detector 140. Besides the use of gaseous detectors in photon counting technique, crystalline Si, CdTe, and CdZnTe can also be used in direct-conversion photon-counting detectors.

This exemplary single photon counting detection technique can discriminate noise in the detector 140 from a true x-ray photon interaction. By counting signals above a predefined threshold, an electronic noise free and efficient counting of single x-ray photons is achieved. When this detector type is used in a slot-scanning system according to embodiments of the application, significant reduction of patient dose and scattered radiation and/or a considerable increase in image quality in terms of contrast and spatial resolution can be obtained, as compared to the integrating detectors (such as direct and indirect flat-panel detectors and CCD devices).

3. Selection of system and grating parameters

Selections of grating parameters and the geometric system parameters in exemplary embodiments can be restricted by the choice of x-ray source, the limitation of the grating fabrication process, the practicality of the system size, the system performance requirements, and the conformation of physical laws. In summary, for a spherical x-ray wave, the system parameters and grating parameters should satisfy the following equations.

1. Spatial coherence requirement

$$\ell_c = \frac{\lambda L}{s} \geq n p_2 \quad , \quad n = 1,2,3,... \tag{3}$$

2. Period of gratings

$$p_0 = \frac{\lambda L}{n p_2} + \sqrt{\left(\frac{\lambda L}{n p_2}\right)^2 + \frac{2\lambda L}{n}} \quad , \quad n = 1,2,3,... \tag{4}$$

$$p_1 = \frac{2 p_0 p_2}{p_0 + p_2} \tag{5}$$

3. Phase grating requirement

The structure height of the silicon phase grating G1 has to be such that the x-rays passing through the grating bars undergo a prescribed phase shift or a phase shift of $\pi$ (as an example), which results in the splitting of the beam into the $\pm$ 1 diffraction orders.

$$h_1 = \frac{\lambda}{2\delta_{Si}} \qquad (6)$$

Also, the structure height of gratings G0 and G2 should be large enough to provide sufficient absorption of x-ray (e.g., > 75%) for selected or optimum system performance.

4. Talbot self-imaging condition

$$d_n = \frac{L\left[\dfrac{\left(n-\dfrac{1}{2}\right)p_1^2}{4\lambda}\right]}{L-\left[\dfrac{\left(n-\dfrac{1}{2}\right)p_1^2}{4\lambda}\right]} \quad , \quad n = 1,2,3,... \qquad (7)$$

The parameters shown in Eqs. (3) - (7) are as follows.

$\ell_c$ = coherence length
$\lambda$ = mean wavelength of x-ray radiation
L = distance between G0 and G1
s = slit width of G0
n = integer (Talbot order)
$d_n$ = Talbot distance between G1 and G2
$p_0$ = period of G0
$p_1$ = period of G1
$p_2$ = period of G2
$h_0$ = structure height of G0
$h_1$ = structure height of G1
$h_2$ = structure height of G2
$\delta_{Si}$ = refractive index decrement of silicon

By first selecting n, $p_2$, $\lambda$, and L based on system requirements and limitations on grating fabrication, other parameters, namely, s, $p_0$, $p_1$, $h_1$, $h_2$, $h_3$, and $d_n$ can then be determined. As an example, Table 1 lists exemplary system design parameters and grating parameters for an embodiment of a slot-scanning phase-contrast digital mammography system.

| TABLE 1 | |
|---|---|
| Mean E (keV) | 28 |
| Mean $\lambda$ (nm) | 0.443 |
| L (mm) | 642 |
| $p_2$ (mm) | 2.0 |
| n | 1 |
| $d_n$ (mm) | 42.4 |
| s (um) | 7 |
| $p_0$ (um) | 30.3 |
| $p_1$ (um) | 3.75 |
| $h_0$ (um) | 42 |

(continued)

| | |
|---|---|
| $h_1$ (um) | 36 |
| $h_2$ (um) | 26 |
| $\ell_c$ (um) | 4.0 |

4. Exemplary system operations

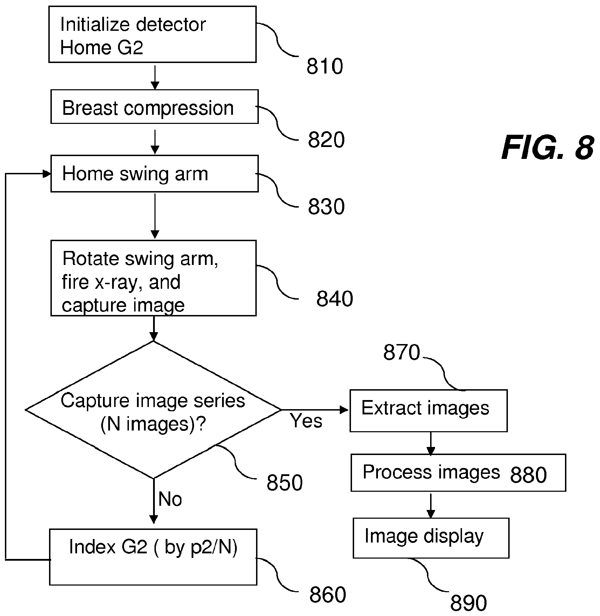

FIG. 8 is a flow chart that shows an embodiment of a method for operating a slot-scanning phase-contrast digital imaging system. The exemplary method embodiment of FIG. 8 will be described using and can be implemented by the system embodiment shown in FIG. 1 and FIG. 3, however the method is not intended to be so limited.

As shown in FIG. 8, after a process starts, the detector is initialized in preparation for exposure and the analyzer grating G2 is moved to a prescribed position or home position (operation block 810). Then, for mammographic medical images, the breast can be compressed (e.g., to improve image quality) (operation block 820). The swing arm 160 is set to an initial or home position (operation block 830). Thus, block 830 can position the x-ray tube 110, the beam shaping assembly 120, the x-ray grating interferometer 130 and the x-ray detector 140 that can be rigidly mounted to the swing arm 160. The x-ray beam can be scanned across the object as the swing arm 160 rotates in an arc like a pendulum covering the width of the object (e.g., about 30 cm) as shown in FIG. 3. When the x-ray beam completes a full scan across the object, the image data recorded by the detector 140 can be read out and stored in a memory unit of a computer (e.g., at the slot-scanning phase-contrast digital imaging system or at a wirelessly coupled control console having a processor, display and memory. In one embodiment, the detector is a long and narrow CCD based detector and can operate in the time delay integration (TDI) mode for signal detection. Then, it is determined whether the image series is complete (e.g., N images have been captured) in operation block 850. When the determination in block 850 is negative, using the phase stepping technique, as an example, the analyzer grating G2 (e.g., mounted on a piezo translation stage) is then moved laterally by a predetermined distance (step) before the next scan of the x-ray beam starts (operation block 860) and the process jumps back to block 830 where the swing arm 160 is returned to the initial pre-scan position or home position (or reversed in rotational direction) to be ready for the next scan in the image series.

When the determination in block 850 is affirmative because a predetermined number of cycles N (e.g., typically 5 to 8) of scanning and stepping are completed, the image data set can be extracted, processed, and displayed on a monitor (operation blocks 870, 880, 890). For example, the same image data set can be processed by an image processing unit of the computer to construct multiple images of the object including absorption contrast, differential phase contrast, phase shift contrast, and dark-field images, as described herein.

These absorption contrast, differential phase contrast, phase shift contrast, and dark-field images are complementary to each other can provide the necessary specificity to visualize subtle details in the object.

There are alternate ways to implement the phase stepping described in the method embodiment of FIG. 8. Exemplary alternate phase stepping implementations include but are not limited to: (i) moving grating G1 (instead of G2) in the direction perpendicular to both the optical axis and the grating bars of G1; (ii) rotating G1 and G2 together around an axis along the orientation of grating bars by an angle (e.g., the two gratings are kept in an aligned position with respect to each other or are fixed together mechanically); or (iii) moving the x-ray source in the direction perpendicular to both the optical axis and the grating bars of the gratings. These exemplary alternate phase stepping implementations can be implemented on the exemplary swing arm 160 configuration shown in FIG. 3.

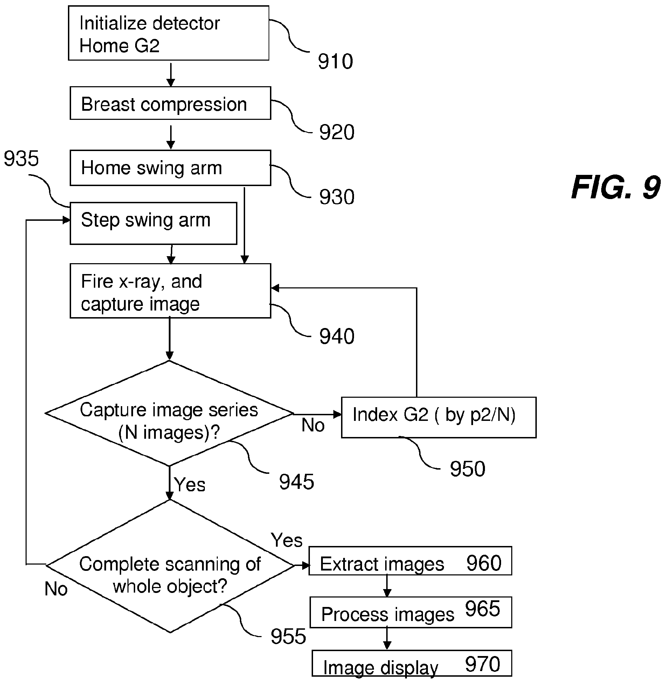

FIG. 9 is a flow chart that shows an embodiment of a method for operating a slot-scanning phase-contrast digital imaging system. The exemplary method embodiment of FIG. 9 will be described using and can be implemented by the system embodiment shown in FIG. 1 and FIGS. 3-4, however the method is not intended to be so limited.

Fig. 9 shows another "step-dither-step" mode of system operations where the swing arm can scan across the object in a step-wise motion. The distance of each step can be about the width of the detector. At each position of the swing arm, a series of x-ray exposure/image capture operations can be performed (e.g., N images captured) using the aforementioned phase stepping technique (e.g., move the analyzer grating G2 by $p_2/N$). Then, the swing arm moves to the next step position and another series of x-ray exposure/image capture operations is performed until the swing arm steps through and completes the whole object scan. Then, the raw image data set is extracted, processed, and displayed on a monitor. Alternatively, as the swing arm steps through the whole object, the raw images data subset can be extracted at the end of each "step", and the captured raw images can be processed and displayed on a monitor concurrently or at the completion of the last step.

As shown in FIG. 9, after a process starts, the detector is initialized in preparation for exposure and the analyzer grating G2 is moved to a prescribed position or home position (operation block 910). Then, an object can be positioned or for mammographic medical images, the breast can be compressed (e.g., to improve image quality) (operation block 920). The swing arm 160 is set to an initial or home position (operation block 930).

Then, the swing arm 160 is stepped to a current step position (operation block 933), the x-ray beam is fired to expose and capture an image of a portion of the object (operation block 940). Then, it is determined whether the image series is complete for that step (e.g., N images have been captured) in operation block 945. When the determination in block 945 is negative, using the phase stepping technique, as an example, the analyzer grating G2 (e.g., mounted on a piezo translation stage) is then moved laterally by a predetermined distance (e.g., $p_2/N$ such as 2mm/8 = 250nm) and the process jumps back to block 940 where the x-ray beam is fired to expose and capture an image of a portion of the object.

When the determination in block 945 is affirmative because a predetermined number of cycles N (e.g., typically 5 to 8) of stepping and scanning are completed, the image data set can be stored and it can be determined in operation block 955 whether scanning is complete for the whole object. When the determination in block 955 is negative, the swing arm 160 is stepped to the next position (operation block 933) and operation blocks 940, 945 and 950 can be repeated. When the determination in block 955 is affirmative because the whole object has been scanned, the image data set can be extracted, processed, and displayed on a monitor (operation blocks 960, 965, 970). For example, the same image data set can be processed by an image processing unit of the computer to construct multiple images of the object including absorption contrast, differential phase contrast, phase shift contrast, and dark-field images, as described herein.

5. Image formation and image retrieval

Without the object in place, the x-ray beam passes through the phase grating G1 and form interference fringes. Having the object in the beam path, the incoming x-ray wavefront is locally distorted by the object causing an angular deviation of the x-ray beam:

$$\alpha(x, y) = \frac{\lambda}{2\pi} \frac{\partial \Phi(x, y)}{\partial x} \qquad (8)$$

Where the wavefront is distorted, these fringes are displaced from their unperturbed position by

$$D(x, y) = d_n \cdot \alpha(x, y) \qquad (9)$$

The fringe displacements are transformed into intensity values by an analyzer grating G2 placed at a distance $d_n$ from the phase grating G1. A two-dimensional detector with much larger pixels than the spacing of the fringes can be used to record the signal. Scanning the lateral position $x_g$ of one of the gratings (e.g., the analyzer grating G2) causes the recorded signal in each pixel to oscillate as a function of $x_g$. For each pixel (i, j), the signal oscillation curve can be expressed by a Fourier series,

$$I_s(i, j, x_g) \approx a_s(i, j) + b_s(i, j) \cos\left(\frac{2\pi}{p_2} x_g + \phi_s(i, j)\right) \qquad (10)$$

(with the object)

$$I_b(i, j, x_g) \approx a_b(i, j) + b_b(i, j) \cos\left(\frac{2\pi}{p_2} x_g + \phi_b(i, j)\right) \qquad (11)$$

(without the object)

From Eqs. (10) and (11), the following images of the object can be retrieved. The transmission image is given by

$$T(i, j) = \frac{a_s(i, j)}{a_b(i, j)} \qquad (12)$$

The differential phase contrast image is given by

$$\left(\frac{\partial \Phi}{\partial x}\right)_{i,j} = \frac{p_2}{\lambda d_n}\left(\phi_s\left(i,j\right) - \phi_b\left(i,j\right)\right) \qquad (13)$$

Also, the phase shift image of the object can be obtained by simple one-dimensional integration along the pixel direction perpendicular to the grating bars, e.g.,

$$\Phi_{i,j} = \frac{p_2}{\lambda d_n}\int\left(\phi_s\left(i,j\right) - \phi_b\left(i,j\right)\right)dx \qquad (14)$$

Furthermore, a dark-field image is formed from higher-angle diffraction intensities scattered by the object. The information about the scattering power of the object is contained in the first Fourier amplitude coefficient, bs(i, j) of Is(i, j, $x_g$). Thus, the dark-field image can be obtained by

$$V\left(i,j\right) = \frac{b_s\left(i,j\right)/a_s\left(i,j\right)}{b_b\left(i,j\right)/a_b\left(i,j\right)} \qquad (15)$$

These four different images of the object can be derived from the same data set and can be complementary to each other to provide multiple information of the object enabling the visualization of subtle details in the object.

As described herein, embodiments of phase-contrast digital imaging systems and/or methods of using the same can provide various advantages according to the application. Embodiments of a hybrid slot-scanning grating-based differential phase contrast mammography system have various advantages (e.g., compared to a full-field digital mammography system).

Embodiments of a grating-based differential phase contrast imaging technique can use conventional x-ray tubes instead of expensive and huge synchrotron radiation sources to provide multiple image information (e.g., absorption contrast image, differential phase contrast image, phase shift image, and dark-field image) of the object from a single image capture process.

Embodiments of slot-scanning grating-based differential phase contrast systems and/or methods can significantly enhance the contrast of low absorbing tissues (e.g., the contrast between healthy and diseased tissues), which can be especially useful for mammography and orthopedic joints.

Embodiments of slot-scanning grating-based differential phase contrast systems and/or methods can allow the use of small gratings and detectors to produce a large-area image. Embodiments can provide reduction in motion blur, scattered radiation, and patient dose without using a grid.

Embodiments of slot-scanning grating-based differential phase contrast systems and/or methods can use a phase grating (G1) and an analyzer grating (G2) with a long and narrow geometry that can be formed by abutting two or more short and narrow (e.g., 8 cm x 1 cm) gratings together and will cost significantly lower than the ones with a large full-field size (24 cm x 30 cm for typical mammography). Thus, embodiments of a tiled detector can be made and will cost much less than a large full-field two-dimensional detector (e.g., 24 cm x 30 cm for typical mammography).

Embodiments of an imaging system can require a long and narrow detector, which can be formed by abutting two or more short and narrow (e.g., 8 cm x 1 cm) detectors together. Smaller detectors with high sensitivity and low noise are commercially available at low cost relative to a large full-field two-dimensional detector (24 cm x 30 cm for typical mammography).

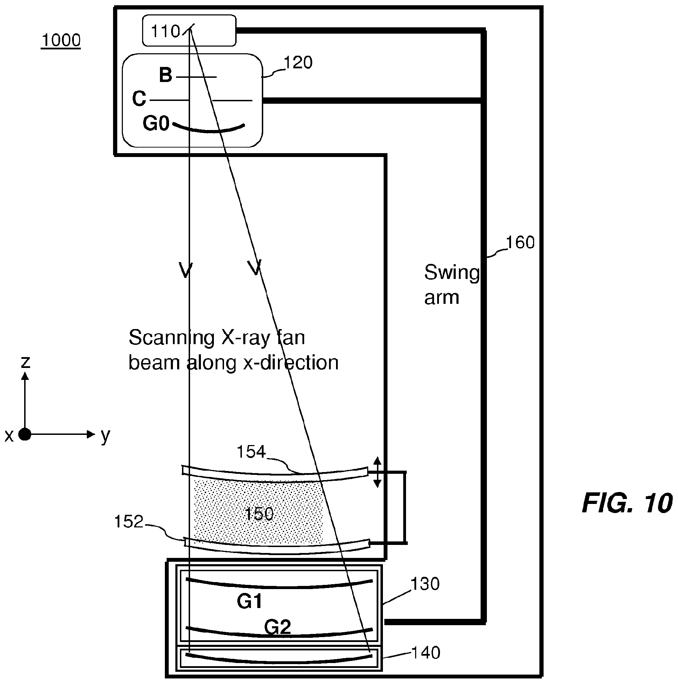

Embodiments of slot-scanning grating-based differential phase contrast systems and/or methods can use curved gratings and detectors circularly around the source focus to enable the design of a more compact system and reduce or eliminate the shadowing effect of the phase grating and/or the scan effect of the analyzer grating occurred in the edge regions of the image. Fig. 10 is a diagram that shows a side view of an embodiment of slot-scanning grating-based differential phase contrast system using curved gratings and detectors that correspond to the x-ray source focus.

Embodiments of slot-scanning grating-based differential phase contrast systems and/or methods can use an x-ray tube with rotating-anode (higher output), a short distance between the x-ray source and the object (higher x-ray fluence), and a detector with a CsI scintillator coupled with a tiled TDI-mode CCD array (higher detection sensitivity). As a result, the exposure time can be significantly reduced.

Certain exemplary embodiments for slot-scanning phase-contrast digital imaging systems and/or methods for using the same, e.g., see Figures 8 and 9, can employ step-dither-step approaches, where one of the gratings, either phase grating G1 or analyzer grating G2, can be stepped with respect to another. For example, when moving analyzer grating G2 where $N$ is a number of steps (e.g., using a piezo translational stage) required to cover a period of grating G2, and the lateral size of the grating G2 is $l_{G2}$; then the scan of an object with lateral size S can use or require $S/l_{G2} \cdot N$ of x-ray exposures. For an exemplary $S$=20 cm breast and 8 phase steps for a 1 cm wide G2 grating at each position (or slice) of the swing arm, then $20/1 \cdot 8$=160 x-ray exposures are used to scan the whole object. Note that $S/l_{G2} \cdot N$ can be considered a sufficient or minimal number needed for a full scan. To properly stitch the slices into an image of the whole object, slight overlaps between slices can be required.

Both exemplary scanning embodiments described in Figures 8 and 9 have either the swing arm or the analyzer grating G2 return back to its initial (e.g., home) position after one slice of the object is scanned. Although, precision positioning of these devices (e.g., translational piezo drivers) can reach the nm scale, the multiple forward-backward types of motions can add up to significant spatial errors after the whole object scan is complete. To reduce or avoid spatial errors, continuous motion of the swing arm with minimal or no stepping of the analyzer grating is preferable. It is also preferable when the relative position of the gratings G1 and G2 does not change (e.g., no stepping) and/or the swing arm continuously moves across the object, which can reduce a scanning time.

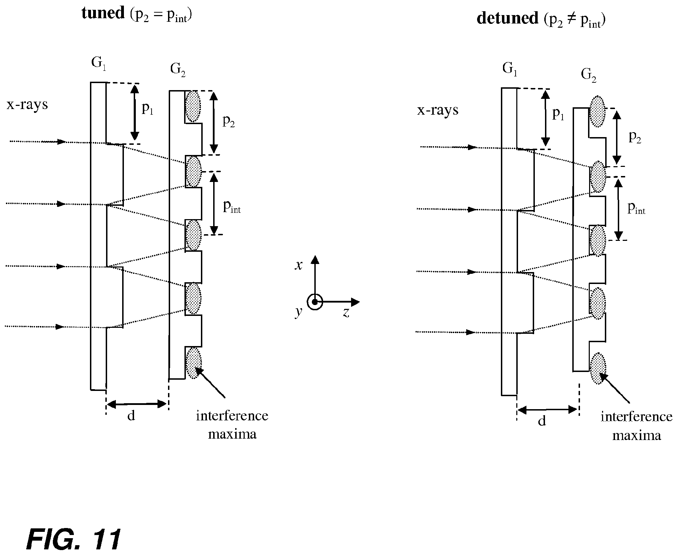

To implement continuous motion of the swing arm with fixed G1 and G2 gratings, exemplary embodiments of phase contrast imaging systems have to be detuned. In one exemplary embodiment, a detuned phase contrast imaging system can be understood to be an imaging system in which the pitch $p_2$ of the analyzer grating G2 is purposely controlled or fabricated to be unequal to a period of interference pattern $p_{int}$ at a Talbot distance behind the phase grating G1. In another exemplary embodiment, a detuned phase contrast imaging system can be understood to be an imaging system in which the pitch $p_2$ of the analyzer grating G2 is controlled or fabricated to be equal to a period of interference pattern $p_{int}$ at a Talbot distance behind the phase grating G1, but the analyzer grating G2 is positioned away from the corresponding Talbot distance. In certain exemplary embodiment, a detuned phase contrast imaging system can generate a periodic fringe pattern, where the fringe pattern occurs over a width or a portion of the width of the analyzer gating G2. Although a number of exposures for detuned grating based PCI system embodiments in a complete or partial scan of an object is about the same, positional errors and/or scanning time can be reduced relative to a tuned grating based PCI systems. Figure 11 is a diagram that illustrates concepts of exemplary tuned and detuned phase contrast imaging systems. The analyzer grating G2 and the interference pattern can be approximated as a cosine waves with the frequencies $f_2 = 1/p_2$ and $f_{int} = 1/p_{int}$, respectively. Then, the signal measured by detector, placed behind the analyzer grating, is:

$$I_s = MTF(f) \cdot [\cos(2\pi f_{int} x) \cdot \cos(2\pi f_2 x)] = \\ MTF(f) \cdot [\cos(2\pi (f_{int} + f_2)x) + \cos(2\pi (f_{int} - f_2)x)]/2. \quad (16)$$

For example, MTF is a detector's modulation transfer function that can be approximated by: $MTF(f) = 0.5 \cdot erfc[\alpha \ln(f/f_0)]$, where $\alpha$ is a slope of the MTF curve and $f_0$ is the spatial frequency at which MTF drops by 50 %. The spatial frequency at $p_2$=2 um pitch of the analyzer grating is 500 cyc/mm. When summed with comparable frequency of interference pattern, it doubles, e.g., $f_{int}+f_2$=1000 cyc/mm. Exemplary values of $f_0$ in indirect charge integrating detectors can be typically between 1 and 2 cyc/mm, while value of $f_0$ can reach 5 cyc/mm in the case of direct photon counting detectors. That said, the detector will measure no signal at 1000 cyc/mm. Therefore, the only detectable signal is:

$$MTF(f) \cdot \cos(2\pi (f_{int} - f_2)x)/2 \quad (17)$$

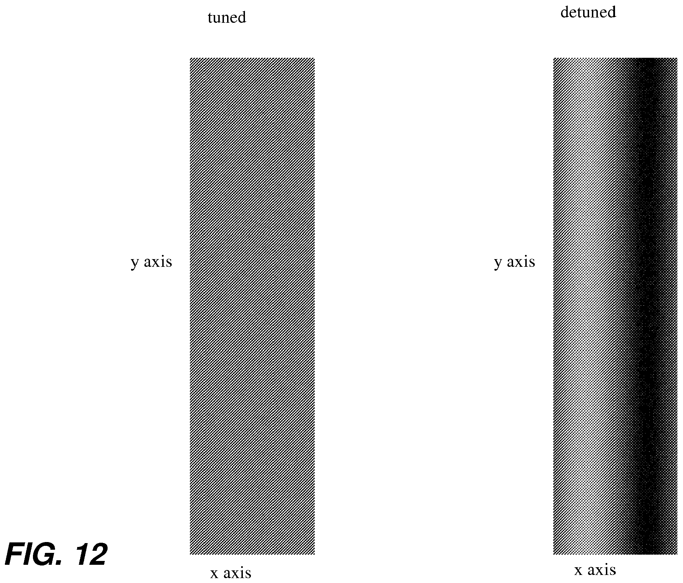

In the case of a tuned phase contrast imaging system ($f_{int} = f_2$), the signal is increased or maximum. When measuring the open field in such configuration, the detector yields the uniform image. In the case of detuned phase contrast imaging system, the detected image will have a cosine pattern with a lower contrast caused by detector's MTF. The loss of the contrast depends on how strongly the system is detuned, i.e. $\Delta f = f_{int} - f_2$. Figure 12 is a diagram that illustrates examples of the open field images measured in the detector plane for tuned and detuned configurations of a phase contrast imaging system embodiment. As shown in FIG. 12, an open field image for a tuned phase contrast imaging system embodiment can produce an unchanging or flat open field image across the analyzer grating G2. As shown in Figure 12, the lateral size of an image is chosen to be equal to one period of fringe pattern as an example. In one embodiment, the phase contrast imaging system, $\Delta f$ can be <5%, <1% or <0.1%.

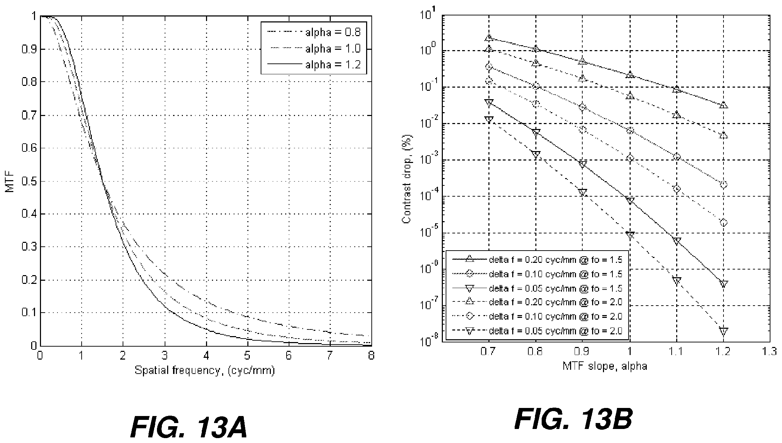

The response of the detector as a function of the spatial frequency is important. FIG. 13A shows several MTFs plotted for different alpha slope (e.g., see equation 16). The MTF with a higher value of slope can have a longer plateau (e.g., slower reduction) for a spatial frequency below the half value frequency. The higher slope is typical for a detector with a better frequency response, for example direct conversion photon-counting detector in comparison to indirect detector. For a case of indirect detectors, the slope $\alpha$ is typically close to 1 and higher, while the half value frequency is in the range between 1.5 and 2 cyc/mm. FIG. 13B shows the percentage of the contrast drop as a function of MTF slope $\alpha$ and spatial frequency $f_0$. As expected, the drop in contrast relative to the maximum possible (e.g., at $\Delta f=0$) is less for smaller $\Delta f$. Also, the curves shown in Figure 13 get even lower for higher $f_0$ (e.g., for a detector with higher quantum efficiency). Higher MTF slope $\alpha$ can further reduce the drop in contrast. The MTF slope $\alpha$ is typically close to 1 and higher. When the PCI system is implemented according to Fig. 3, the width of G2 grating can be selected based on $\Delta f$. If the width of G2 is set to be equal to one period of the measured fringe pattern, then for $\Delta f=0.20$, 0.10, or 0.05 cyc/mm the width of G2 can be 0.5, 1, or 2 cm, respectively. As described herein, to avoid the non-uniformity in grating fabrication, it is preferable to keep the width of the analyzer grating small. Therefore, the width of 1 cm with corresponding $\Delta f=0.1$ cyc/mm can be the most suitable, although, embodiments of the application are not intended to be so limited. Further, other sizes can be used when the width of G2 is equal to not one but two or more periods of interferometeric contrast.

In contrast to embodiments of tuned phase contrast imaging systems, embodiments of detuned system can only be implemented according to schematics shown in Figure 3. The fringe patters in the detector plane has to be oriented such that the arms swings laterally across the pattern. While PCI implementation depicted on Figure 4 is suitable for tuned phase contrast imaging system, it cannot be applied to detuned PCI system. Additionally, in case of embodiments of detuned PCI systems, the analyzer grating G2 and the detector D can be moved together (e.g., using an attached translational piezo driver) to simultaneously move them in the direction of the x-ray beam (e.g., $z$ axis) such that the frequency ($\Delta f$) of fringe pattern in the detector plane can be adjusted.

When the width of the analyzer grating G2 is chosen, for example 1 cm, it might be challenging to precisely fabricate the grating with the pitch that would form expected frequency of the fringe pattern at the detector plane, for example 0.1 cyc/mm. In one embodiment, when the pitch G2 is slightly off of the desired or selected dimensions, the phase contrast imaging system can be tweaked by shifting the analyzer grating G2 along the beam axis (e.g., axis z) relative to the phase grating G1. By shifting the analyzer grating G2 along the beam axis, the analyzer grating G2 can peak at different z position of the interference pattern formed by phase grating G1. In other words, in certain exemplary embodiments, the different frequency of interference pattern, $f_{int}$, is used to form the desired fringe pattern at the detector plane.

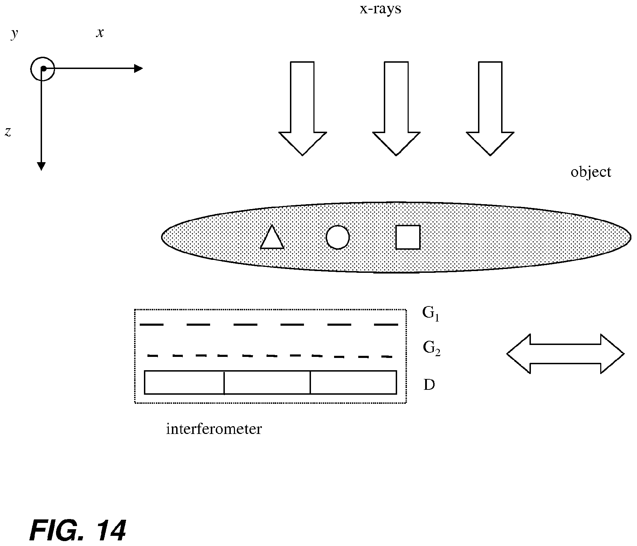

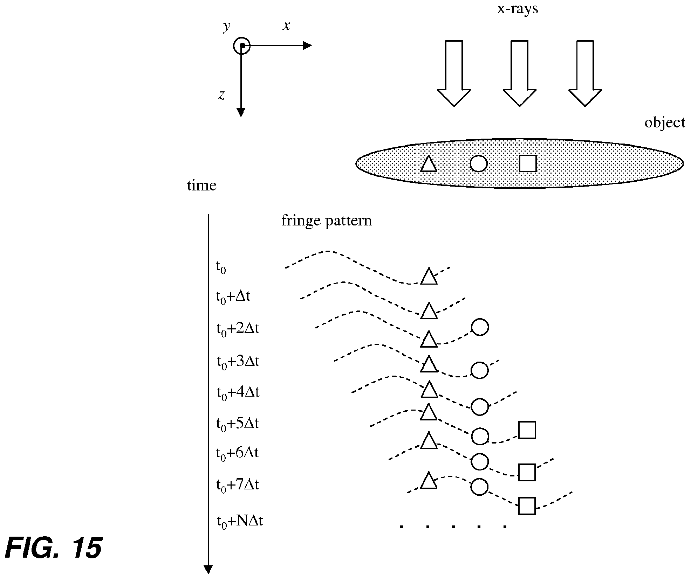

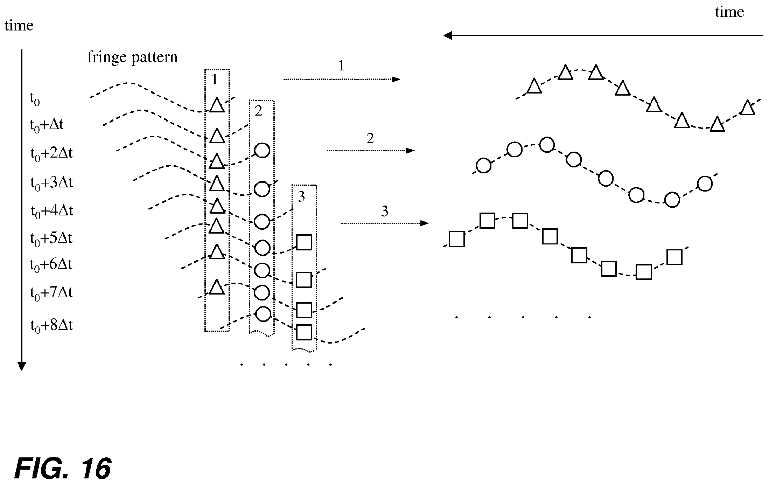

As described herein, in embodiments of tuned phase contrast imaging systems, the phase retrieval algorithm can require multiple x-ray exposures at different lateral positions of analyzer grating, which allows forming a cosine shaped intensity curve shown in Figure 7. When the phase contrast imaging system is detuned, the detector can already measure the cosine shaped fringe pattern and the grating stepping is no longer required. Instead, in some exemplary embodiments, the grating G1, the grating G2 and the detector D can be fixed at one relative position and moved to image the object, for example attached to a swing arm, and the swing arm can be continuously moved across the stationary object. Alternatively, in one embodiment, the swing arm can be at rest and the object can be laterally moved across in the plane perpendicular to incident x-rays. Figure 14 is a diagram that illustrates exemplary motion of interferometer with respect to objects or vise versa for a phase contrast imaging system embodiment. Figure 15 is a diagram that illustrates exemplary of object scan schematics that project individual slices of the object onto one-period fringe pattern measured in the detector plane. Triangle, circle, and square shapes shown in Figures 14-15 refer to different parts of the exemplary object. When the object and the swing arm with fixed G1, G2, and D are moved relative to each other, those object parts are individually projected on different lateral positions of the fringe pattern at subsequent instances of time. After, the scan of the whole object is completed, each individual part of the object, such as triangle, circle and square, is measured several times (e.g., $N=8$) at different intensity. In other words, each of the exemplary shapes (e.g., triangle, circle, and square) will have their individual intensity curve similar to the one shown in Figure 7. Figure 16 shows the schematics of intensity curve formation for an individual slice of the object (e.g., triangles, circles, and squares). Again, the Fourier based reconstruction technique, described herein, can be applied to each of the intensity curves to form the transmission, differential phase, and dark-field images for each of the slices. Then the slice images can be combined or stitched together to form image(s) of the full object.

The functional diagram in Figure 2 drawn for a case of a tuned PCI system can also be applied to detuned PCI system. However, for a detuned PCI system embodiment, the piezo translational stage is not required, since the grating is no longer stepped in the detuned PCI configuration.

In accordance with certain exemplary embodiments, there can be provided methods that can include providing an x-ray generator for radiographic imaging, providing a beam shaping assembly comprising a beam limiting apparatus and a source grating G0, providing an x-ray grating interferometer comprising a phase grating G1, and an analyzer grating G2, and offsetting a pitch of the analyzer grating G2 relative to a pitch of an interference pattern produced by the phase grating G1 at a prescribed distance from the phase grating G1. In one method embodiment, the relative position of the phase gratings G1 and the analyzer grating G2 does not change for a scan of an object, and where the prescribed distance is a Talbot distance. One method embodiment can include producing a fringe pattern that is greater than 0.1 cm or over a significant portion of the analyzer grating G2. In one method embodiment, the grating G1, the grating G2 and the detector D can be fixed at one relative position, attached to the swing arm and moved to image the object, where the relative position of the grating G1 and the grating G2 provide a non-zero $\Delta f$. In one method embodiment, a fringe pattern is produced by the pitch of the analyzer grating G2 being unequal to the pitch of an interference pattern produced by the phase grating G1 at a position of the analyzer grating G2, or the fringe pattern is produced by the position of the analyzer grating G2 being offset from a Talbot distance when the pitch of the analyzer grating G2 is equal to a pitch of the interference pattern.

Embodiments of slot-scanning grating-based differential phase contrast systems and/or methods can provide a wide range of potential applications including medical imaging, small-animal imaging, security screening, industrial non-destructive testing, and food inspection. Embodiments according to the application can also be used for phase-contrast applications using other forms of radiation such as neutron and atom beams. Embodiments according to the application can provide a robust and low-cost phase-contrast mammography system with high efficiency and large field of view for clinical applications.

Further, when embodiments according to the application (e.g., grating-based PCI) are combined with a tomographic scan, the three-dimensional distribution of x-ray refraction index in the object as well as the distribution of absorption coefficient commonly obtained in absorption tomography can be reconstructed.

While the invention has been illustrated with respect to one or more implementations, alterations and/or modifications can be made to the illustrated examples without departing from the spirit and scope of the appended claims. In addition, while a particular feature of the invention can have been disclosed with respect to only one of several implementations, such feature can be combined with one or more other features of the other implementations as can be desired and advantageous for any given or particular function. The term "at least one of' is used to mean one or more of the listed items can be selected. The term "about" indicates that the value listed can be somewhat altered, as long as the alteration does not result in nonconformance of the process or structure to the illustrated embodiment. Finally, "exemplary" indicates the description is used as an example, rather than implying that it is an ideal. Other embodiments of the invention will be apparent to those skilled in the art from consideration of the specification and practice of the invention disclosed herein.

**Claims**

1. A phase-contrast digital radiographic imaging system (100) comprising:

   a polychromatic x-ray source (110) for radiographic imaging;
   a beam shaping assembly (120) comprising a source grating G0 and a collimator (C), the collimator (C) configured to provide a long and narrow fan shaped x-ray beam having a beam length and a beam width,
   an x-ray grating interferometer (130) comprising
   a phase grating G1, and
   an analyzer grating G2, wherein the phase grating G1 and the analyzer grating G2 are long and narrow and are covered by the beam length and the beam width; and
   an area x-ray detector (140) to capture a radiographic image of an object (150) placed between the source grating G0 and the phase grating G1, the x-ray detector (140) having a long and narrow active area comprising an active length and an active width that are covered by the beam length and the beam width,
   the x-ray source (110), the source grating G0, the phase grating G1, the analyzer grating G2, and the detector (140) are rigidly mounted to a swing arm (160), the swing arm (160) configured to pivot about an axis at the x-ray source (110) to move the x-ray source (110), the source grating G0, the phase grating G1, the analyzer grating G2, and the detector (140) about the axis to scan across the object (150) while the object (150) is in a stationary position, and
   where a pitch and a position of the analyzer grating G2 relative to a pitch of an interference pattern produced by the phase grating G1 produce at least one fringe pattern over a width of the analyzer grating G2.

2. The system (100) of claim 1, where the fringe pattern is produced by the pitch of the analyzer grating G2 being unequal to the pitch of an interference pattern produced by the phase grating G1 at a position of the analyzer grating G2, where the analyzer grating G2 is at a Talbot distance or a position to increase contrast at the position of the detector (140), where the relative position of the phase grating G1 and the analyzer grating G2 does not change for a scan of an object (150).

3. The system (100) of claim 1, where the fringe pattern is produced by the position of the analyzer grating G2 is offset from a Talbot distance, and where the pitch of the analyzer grating G2 is equal to a pitch of the interference pattern.

4. The system (100) of claim 1, where the phase-contrast digital radiographic imaging system (100) is detuned, and where the relative position of the phase gratings G1 and the analyzer grating G2 does not change for a complete scan of an object (150).

5. The system (100) of claim 1, where the phase-contrast digital radiographic imaging system (100) is detuned to produce a fringe pattern that is greater than 0.1 cm, or over a significant portion of the analyzer grating G2.

6. The system (100) of claim 1, where *N* is a number of steps used to cover a period of the analyzer grating G2, and where a scan of the object (150) includes N exposures of at least one portion of the object (150).

7. The system (100) of claim 1, where the grating G1, the grating G2 and a detector D (140) can be fixed at one relative position, attached to the swing arm (160) and moved to image the object (150), where the relative position of the grating G1 and the grating G2 provide a non-zero $\Delta f = f_{int} - f_2$, where a frequency ($\Delta f$) of fringe pattern at the detector (140) can be adjusted, where the analyzer grating G2 can be moved offset relative to the phase grating G1 to adjust the frequency ($\Delta f$) of fringe pattern at the detector (140), or the analyzer grating G2 and the detector D (140) can be moved simultaneously together in the direction of the x-ray beam to increase a contrast or adjust the fringe pattern at the detector (140).

8. The system (100) of claim 1, where the phase-contrast DR imaging system (100) is a slot-scanning phase-contrast DR imaging system (100), where an orientation of the grating bars of the three gratings G0, G1, and G2 is parallel to the scan direction of the swing arm (160), where an image data set from a single pass of the system (100) over an object (150) is used to construct multiple images of the object (150) including at least one of absorption contrast images, differential phase contrast images, phase shift contrast images, and dark-field images.

9. The system (100) of claim 1,
where the pitch of the analyzer grating G2 relative to the pitch of the interference pattern produced by the phase grating G1 are unequal at a position of the area x-ray detector (140), where the relative position of the phase gratings G1 and the analyzer grating G2 does not change for a scan of an object (150), where *N* is a number of steps used to cover a period of the analyzer grating G2, and where the scan of the object (150) includes N exposures of at least one portion of the object (150).

10. The system (100) of claim 1, wherein the three gratings (G0, G1, and G2) are positioned in such a way that the plane and the grating bars of these gratings are aligned parallel to each other.

11. The system (100) of claim 1 or 10, where for each of a plurality of N positions of the analyzer grating G2, the swing arm (160) performs a single FOV scan by exposing M sequential positions of the area x-ray detector (140) to obtain a plurality of raw image data used to construct a 3D image, where M and N are positive integers greater than 4, where the M sequential positions of the area x-ray detector (140) are a plurality of adjacent overlapping positions, where gratings of the analyzer grating G2 are substantially parallel or substantially perpendicular to an x-ray beam path.

12. The system (100) of claim 10, where for each of a plurality of N positions of the analyzer grating G2, the swing arm (160) performs a M FOV scans by continuously slot- scanning M portions of the FOV using a CCD detector (140) in TDI mode to obtain a plurality of raw image data used to construct a 3D image, where M and N are positive integers, where the M portions of the FOV are sequentially adjacent positions of the FOV, where gratings of the analyzer grating G2 are substantially parallel to an x-ray beam path.

13. The system (100) of claim 10, where the swing arm (160) performs a single FOV scan by exposing M sequential positions of the area x-ray detector (140), where each of the M sequential positions are exposed for each of a plurality of N positions of the analyzer grating G2 to obtain a plurality of raw image data used to construct a 3D image, where M and N are positive integers, where the M sequential positions of the area x-ray detector (140) are a plurality of adjacent overlapping positions, where gratings of the analyzer grating G2 are substantially parallel or substantially perpendicular to an x-ray beam path.

14. The system (100) of claim 10, further comprising a filter, where the detector (140) is an indirect area detector (140) or a direct area detector (140), where the gratings and detector (140) are curved to match the source x-ray focus, where the phase grating G1 and the analyzer grating G2 have a prescribed angle therebetween, where the analyzer grating G2 can be stepped linearly or stepped rotationally, where an aspect ratio of the second grating and the third grating are each greater than 2:1.

**Patentansprüche**

1.  Digitales Phasenkontrast-Röntgenbildgebungssystem (100), welches Folgendes aufweist:

    eine polychromatische Röntgenstrahlquelle (110) zur Röntgenbildgebung;
    eine Strahlformungsanordnung (120), die ein Quellengitter G0 und einen Kollimator (C) aufweist, wobei der Kollimator (C) konfiguriert ist, um einen langen und schmalen fächerförmigen Röntgenstrahl mit einer Strahllänge und einer Strahlbreite vorzusehen,
    ein Röntgengitter-Interferometer (130), welches Folgendes aufweist:

    ein Phasengitter G1, und
    ein Analysatorgitter G2, wobei das Phasengitter G1 und das Analysatorgitter G2 lang und schmal sind und durch die Strahllänge und die Strahlbreite abgedeckt sind; und
    einen Flächen-Röntgendetektor (140) zum Aufnehmen eines Röntgenbildes eines Objektes (150), welches zwischen dem Quellengitter G0 und dem Phasengitter G1 angeordnet ist, wobei der Röntgendetektor (140) eine lange und schmale aktive Fläche hat, welche eine aktive Länge und eine aktive Breite hat, die durch die Strahllänge und die Strahlbreite abgedeckt sind,
    wobei die Röntgenquelle (110), das Quellengitter G0, das Phasengitter G1, das Analysatorgitter G2 und der Detektor (140) starr an einem Schwenkarm (160) montiert sind, wobei der Schwenkarm (160) zum Schwenken um eine Achse bei der Röntgenquelle (110) konfiguriert ist, um die Röntgenquelle (110), das Quellengitter G0, das Phasengitter G1, das Analysatorgitter G2 und den Detektor (140) um die Achse zu bewegen, um über das Objekt (150) zu scannen bzw. zu streichen, während das Objekt (150) in einer stationären Position ist, und
    wobei eine Teilung und eine Position des Analysatorgitters G2 relativ zu einer Teilung eines Interferenzmusters, welches durch das Phasengitter G1 erzeugt wird, zumindest ein Streifenmuster über eine Breite des Analysatorgitters G2 erzeugen.

2.  System (100) nach Anspruch 1, wobei das Streifenmuster, welches durch die Teilung des Analysatorgitters G2 erzeugt wird, ungleich der Teilung eines Interferenzmusters ist, welches durch das Phasengitter G1 an einer Position des Analysatorgitters G2 erzeugt wird, wobei das Analysatorgitter G2 bei einer Talbot-Distanz oder einer Position ist, um den Kontrast an der Position des Detektors (140) zu vergrößern, wobei die relative Position des Phasengitters G1 und des Analysatorgitters G2 sich für eine Abtastung eines Objektes (150) nicht ändert.

3.  System (100) nach Anspruch 1, wobei das Streifenmuster, welches durch die Position des Analysatorgitters G2 erzeugt wird, von einer Talbot-Distanz versetzt bzw. entfernt ist, und wobei die Teilung des Analysatorgitters G2 gleich einer Teilung des Interferenzmusters ist.

4.  System (100) nach Anspruch 1, wobei das digitale Phasenkontrast-Röntgenbildgebungssystem (100) verstimmt bzw. fehleingestellt wird, und wobei die relative Position des Phasengitters G1 und des Analysatorgitters G2 sich für eine vollständige Abtastung eines Objektes (50) nicht verändert

5.  System (100) nach Anspruch 1, wobei das digitale Phasenkontrast-Röntgenbildgebungssystem (100) verstimmt bzw. fehleingestellt wird, um ein Streifenmuster zu erzeugen, welches größer als 0,1 cm ist, oder über einen signifikanten Teil des Analysatorgitters G2 geht.

6.  System (100) nach Anspruch 1, wobei N eine Anzahl von Schritten ist, die verwendet wird, um eine Periode des Analysatorgitters G2 abzudecken, und wobei eine Abtastung des Objektes (150) N Belichtungen von mindestens einem Teil des Objektes (150) aufweist.

7.  System (100) nach Anspruch 1, wobei das Gitter G1, das Gitter G2 und ein Detektor D (140) an einer Relativposition fixiert sein können, an dem Schwenkarm (160) angebracht werden können und bewegt werden können, um das Objekt (150) abzubilden, wobei die relative Position des Gitters G1 und des Gitters G2 ein $\Delta f = f_{nt} - f_2$ von ungleich null vorsehen, wobei eine Frequenz ($\Delta f$) des Streifenmusters beim Detektor (140) eingestellt werden kann, wobei das Analysatorgitter G2 relativ zum Phasengitter G1 versetzt bewegt werden kann, um die Frequenz ($\Delta f$) des Streifenmusters beim Detektor (140) einzustellen, oder wobei das Analysatorgitter G2 und der Detektor D (140) gleichzeitig zusammen in der Richtung des Röntgenstrahls bewegt werden können, um einen Kontrast zu vergrößern oder das Streifenmuster beim Detektor (140) einzustellen.

8. System (100) nach Anspruch 1, wobei das digitale Phasenkontrast-Röntgenbildgebungssystem (100) ein digitales Phasenkontrast-Röntgenbildgebungssystem (100) mit Schlitzscan bzw. Schlitzabtastung ist, wobei eine Orientierung der Gitterbalken der drei Gitter G0, G1 und G2 parallel zur Abtastungsrichtung des Schwenkarms (160) ist, wobei ein Bilddatensatz von einem einzigen Durchgang des Systems (100) über ein Objekt (150) verwendet wird, um mehrere Bilder des Objektes (150) zusammenzusetzen, welche zumindest eines der Folgenden aufweisen: Absorptionskontrastbilder, Differenzphasenkontrastbilder, Phasenverschiebungskontrastbilder und Dunkelfeldbilder.

9. System (100) nach Anspruch 1, wobei die Teilung des Analysatorgitters G2 relativ zur Teilung des Interferenzmusters, welches durch das Phasengitter G1 erzeugt wird, an einer Position des Flächen-Röntgendetektors (140) ungleich sind, wobei die relative Position des Phasengitters G1 und des Analysatorgitters G2 sich für eine Abtastung eines Objektes (150) nicht verändert, wobei N eine Anzahl von Schritten ist, die verwendet wird, um eine Periode des Analysatorgitters G2 abzudecken, und wobei die Abtastung des Objektes (150) N Belichtungen von mindestens einem Teil des Objektes (150) aufweist.

10. System (100) nach Anspruch 1, wobei die drei Gitter (G0, G1 und G2) derart positioniert sind, dass die Ebene und die Gitterbalken dieser Gitter parallel zueinander angeordnet sind.

11. System (100) nach Anspruch 1 oder 10, wobei für jede der Vielzahl von N Positionen des Analysatorgitters G2, der Schwenkarm (160) eine einzige FOV-Abtastung ausführt, und zwar durch Belichten von M sequentiellen Positionen des Flächen-Röntgendetektors (140), um eine Vielzahl von Rohbilddaten zu erhalten, die verwendet werden, um ein dreidimensionales Bild zusammenzusetzen, wobei M und N positive ganze Zahlen größer als 4 sind, wobei die M sequentiellen Positionen des Flächen-Röntgendetektors (140) eine Vielzahl von benachbarten überlappenden Positionen sind, wobei Gitter des Analysatorgitters G2 im Wesentlichen parallel oder im Wesentlichen senkrecht zu einem Röntgenstrahlpfad sind.

12. System (100) nach Anspruch 10, wobei für jede der Vielzahl von N Positionen des Analysatorgitters G2 der Schwenkarm (160) M FOV-Abtastungen durch kontinuierliches Abtasten mit einem Schlitz- bzw. Slot-Scanning von M Teilen des FOV unter Verwendung eines CCD-Detektors (140) im TDI-Modus ausführt, um eine Vielzahl von Rohbilddaten zu erhalten, die verwendet werden, um ein dreidimensionales Bild aufzubauen, wobei M und N positive ganze Zahlen sind, wobei M Teile des FOV sequentiell benachbarte Positionen des FOV sind, wobei Gitterelemente des Analysatorgitters G2 im Wesentlichen parallel zu einem Röntgenstrahlpfad sind.

13. System (100) nach Anspruch 1, wobei der Schwenkarm (160) eine einzige FOV-Abtastung ausführt durch Belichten von M aufeinanderfolgenden Positionen des Flächen-Röntgendetektors (140), wobei jede der M aufeinanderfolgenden Positionen für jeweils eine Vielzahl von N Positionen des Analysatorgitters G2 belichtet werden, um eine Vielzahl von Rohbilddaten zu erhalten, die verwendet werden, um ein dreidimensionales Bild zusammenzusetzen, wobei M und N positive ganze Zahlen sind, wobei die M aufeinanderfolgenden Positionen des Flächen-Röntgendetektors (140) eine Vielzahl von benachbarten überlappenden Positionen sind, wobei Gitterelemente des Analysatorgitters G2 im Wesentlichen parallel oder im Wesentlichen senkrecht zu einem Röntgenstrahlpfad sind.

14. System (100) nach Anspruch 1, welches weiter einen Filter aufweist, wobei der Detektor (140) ein indirekter Flächendetektor (140) oder ein direkter Flächendetektor (140) ist, wobei die Gitter und der Detektor (140) gekrümmt sind, um zu dem Röntgenstrahlfokus zu passen, wobei das Phasengitter G1 und das Analysatorgitter G2 einen vorgeschriebenen Winkel dazwischen haben, wobei das Analysatorgitter G2 linear gestuft sein kann oder in Drehrichtung gestuft sein kann, wobei ein Seitenverhältnis des zweiten Gitters und des dritten Gitters jeweils größer als 2:1 sind.

**Revendications**

1. Système d'imagerie radiographique numérique à contraste de phase (100) comprenant :

    une source de rayons X polychromatique (110) pour de l'imagerie radiographique ;
    un ensemble de mise en forme de faisceau (120) comprenant un réseau de source G0 et un collimateur (C), le collimateur (C) étant agencé pour fournir un faisceau de rayons X en forme d'éventail long et étroit ayant une longueur de faisceau et une largeur de faisceau,
un interféromètre de rayons X à réseaux (130) comprenant
un réseau de phase G1, et
un réseau d'analyseur G2, le réseau de phase G1 et le réseau d'analyseur G2 étant longs et étroits et étant couverts par la longueur de faisceau et la largeur de faisceau ; et
un détecteur de rayons X de région (140) pour capturer une image radiographique d'un objet (150) placé entre le réseau de source G0 et le réseau de phase G1, le détecteur de rayons X (140) ayant une région active longue et étroite comprenant une longueur active et une largeur active qui sont couvertes par la longueur de faisceau et la largeur de faisceau,
la source de rayons X (110), le réseau de source G0, le réseau de phase G1, le réseau d'analyseur G2 et le détecteur (140) sont montés de manière rigide sur un bras oscillant (160), le bras oscillant (160) étant agencé pour pivoter autour d'un axe au niveau de la source de rayons X (110) pour déplacer la source de rayons X (110), le réseau de source G0, le réseau de phase G1, le réseau d'analyseur G2 et le détecteur (140) autour de l'axe pour balayer l'objet (150) pendant que l'objet (150) est dans une position fixe, et
dans lequel un pas et une position du réseau d'analyseur G2 par rapport à un pas d'un motif d'interférences produit par le réseau de phase G1 produisent au moins un motif de franges sur une largeur du réseau d'analyseur G2.

2. Système (100) selon la revendication 1, dans lequel le motif de franges est produit par le fait que le pas du réseau d'analyseur G2 n'est pas égal au pas d'un motif d'interférence produit par le réseau de phase G1 à une position du réseau d'analyseur G2, dans lequel le réseau d'analyseur G2 est à une distance de Talbot ou à une position permettant d'augmenter le contraste au niveau de la position du détecteur (140), la position relative du réseau de phase G1 et du réseau d'analyseur G2 ne changeant pas pour l'analyse d'un objet (150).

3. Système (100) selon la revendication 1, dans lequel le motif de franges est produit par le fait que la position du réseau d'analyseur G2 est décalée par rapport à une distance de Talbot, et dans lequel le pas du réseau d'analyseur G2 est égal au pas du motif d'interférence.

4. Système (100) selon la revendication 1, dans lequel le système d'imagerie radiographique numérique à contraste de phase (100) est déréglé, et dans lequel la position relative du réseau de phase G1 et du réseau d'analyseur G2 ne change pas dans une analyse complète d'un objet (150).

5. Système (100) selon la revendication 1, dans lequel le système d'imagerie radiographique numérique à contraste de phase (100) est déréglé pour produire un motif de franges qui est supérieur à 0,1 cm, ou sur une portion significative du réseau d'analyseur G2.

6. Système (100) selon la revendication 1, dans lequel N est un nombre d'étapes utilisées pour couvrir une période du réseau d'analyseur G2, et dans lequel une analyse de l'objet (150) comprend N expositions d'au moins une portion de l'objet (150).

7. Système (100) selon la revendication 1, dans lequel le réseau G1, le réseau G2 et un détecteur D (140) peuvent être fixés à une position relative, attachés au bras oscillant (160) et déplacés pour capturer l'image de l'objet (150), dans lequel la position relative du réseau G1 et du réseau G2 fournit un $\Delta f = f_{int} - f_2$ non nul, dans lequel une fréquence ($\Delta f$) de motif de franges au niveau du détecteur (140) peut être ajustée, dans lequel le réseau d'analyseur G2 peut être déplacé décalé par rapport au réseau de phase G1 pour ajuster la fréquence ($\Delta f$) du motif de franges au niveau du détecteur (140), ou le réseau d'analyseur G2 et le détecteur D (140) peuvent être déplacés simultanément ensemble dans la direction du faisceau de rayons X pour augmenter le contraste ou ajuster le motif de franges au niveau du détecteur (140) .

8. Système (100) selon la revendication 1, dans lequel le système d'imagerie DR à contraste de phase (100) est un système d'imagerie DR à contraste de phase à balayage de fente (100), dans lequel l'orientation des barres de réseau des trois réseaux G0, G1 et G2 est parallèle à la direction de balayage du bras oscillant (160), dans lequel un ensemble de données d'images provenant d'une seule passe du système (100) sur un objet (150) est utilisé pour construire de multiples images de l'objet (150) y compris au moins une sorte d'images parmi des images de contraste d'absorption, des images de contraste de phase différentielle, des images de contraste de décalage de phase, et des images de fond noir.

**9.** Système (100) selon la revendication 1, dans lequel le pas du réseau d'analyseur G2 et le pas du motif d'interférences produit par le réseau de phase G1 sont inégaux à une position du détecteur de rayons X de région (140), dans lequel la position relative des réseaux de phase G1 et du réseau d'analyseur G2 ne change pas pour le balayage d'un objet (150), dans lequel N est un nombre de pas utilisés pour couvrir une période du réseau d'analyseur G2, et dans lequel le balayage de l'objet (150) comprend N expositions d'au moins une portion de l'objet (150).

**10.** Système (100) selon la revendication 1, dans lequel les trois réseaux (G0, G1 et G2) sont disposés de telle sorte que le plan et les barres de ces réseaux sont alignés parallèles entre eux.

**11.** Système (100) selon la revendication 1 ou 10, dans lequel pour chacune d'une pluralité de N positions du réseau d'analyseur G2, le bras oscillant (160) réalise un seul balayage FOV en exposant M positions séquentielles du détecteur de rayons X de région (140) pour obtenir une pluralité de données d'images brutes utilisées pour construire une image 3D, M et N étant des entiers positifs supérieurs à 4, les M positions séquentielles du détecteur de rayons X de région (140) étant une pluralité de positions adjacentes en chevauchement, les barres du réseau d'analyseur G2 étant sensiblement parallèles ou sensiblement perpendiculaires à un trajet de faisceau de rayons X.

**12.** Système (100) selon la revendication 10, dans lequel pour chacune d'une pluralité de N positions du réseau d'analyseur G2, le bras oscillant (160) réalise M balayages FOV en analysant par fente en continu M portions du FOV en utilisant un capteur CCD (140) en mode TDI pour obtenir une pluralité de données d'images brutes utilisées pour construire une image 3D, M et N étant des entiers positifs, les M portions du FOV étant des positions séquentiellement adjacentes du FOV, les barres du réseau d'analyseur G2 étant sensiblement parallèles à un trajet de faisceau de rayons X.

**13.** Système (100) selon la revendication 10, dans lequel le bras oscillant (160) réalise un seul balayage FOV en exposant M positions séquentielles du détecteur de rayons X de région (140), chacune des M positions séquentielles étant exposée pour chacune d'une pluralité de N positions du réseau d'analyseur G2 pour obtenir une pluralité de données d'images brutes utilisées pour construire une image 3D, M et N étant des entiers positifs, les M positions séquentielles du détecteur de rayons X de région (140) étant une pluralité de positions adjacentes en chevauchement, les barres du réseau d'analyseur G2 étant sensiblement parallèles ou sensiblement perpendiculaires à un trajet de faisceau de rayons X.

**14.** Système (100) selon la revendication 10, comprenant en outre un filtre, dans lequel le détecteur (140) est un détecteur de région indirect (140) ou un détecteur de région direct (140), dans lequel les réseaux et le détecteur (140) sont courbes pour concorder avec le foyer la source de rayons X, dans lequel le réseau de phase G1 et le réseau d'analyseur G2 font un angle prescrit entre eux, dans lequel le réseau d'analyseur G2 peut être déplacé pas à pas linéairement ou de façon rotative, dans lequel chacun des facteurs de forme du deuxième réseau et du troisième réseau est supérieur à 2:1.

FIG. 1

Scanning X-ray fan beam along x-direction

Swing arm

EP 2 830 505 B1

**FIG. 2**

EP 2 830 505 B1

Collimator (slot)

scan direction
(e.g., across the breast)

G0

object scanned
(e.g., breast (fixed in position)

G1

24 cm

Phase stepping after
each complete scan

G2

Pixel (i, j)

Detector

1 cm

*FIG. 3*

EP 2 830 505 B1

X-ray
focal spot

scan direction
(e.g., across the breast)

Collimator (slot)

G0

object scanned
(e.g., breast (fixed in position)

G1

24 cm

FIG. 4

G2

Phase stepping after
each complete scan

Pixel (i, j)

Detector

1 cm

*FIG. 5*

**FIG. 6A**

**FIG. 6B**

*FIG. 7*

**FIG. 8**

Initialize detector Home G2 — 810

Breast compression — 820

Home swing arm — 830

Rotate swing arm, fire x-ray, and capture image — 840

Capture image series (N images)? — 850

Yes → Extract images — 870 → Process images 880 → Image display — 890

No → Index G2 ( by p2/N) — 860

FIG. 9

FIG. 10

1000

110

B

C

G0

120

Swing arm

160

Scanning X-ray fan beam along x-direction

z

x

y

154

150

152

G1

G2

130

140

EP 2 830 505 B1

30

**tuned** $(p_2 = p_{int})$

**detuned** $(p_2 \neq p_{int})$

FIG. 11

EP 2 830 505 B1

detuned

tuned

y axis

y axis

x axis

x axis

*FIG. 12*

FIG. 13B

FIG. 13A

x-rays

object

G₁

G₂

D

interferometer

*FIG. 14*

EP 2 830 505 B1

34

*FIG. 15*

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007183583 A1
- US 2009092227 A1